# EUROPEAN PATENT SPECIFICATION

(11) **EP 4 434 355 B1**
(45) Date of publication and mention of the grant of the patent: **22.07.2026**
(21) Application number: 22931726.8
(22) Date of filing: 13.09.2022
(51) Int. Cl.: A23K 10/12, A23K 10/14, A23K 10/30, A23L 27/30, A23L 2/60, A23L 33/105, C07J 17/00, C08L 5/00

(54) **SWEETENING AGENT COMPOSITION, PREPARATION METHOD THEREFOR AND USE THEREOF**
SÜSSUNGSMITTELZUSAMMENSETZUNG, HERSTELLUNGSVERFAHREN DAFÜR UND VERWENDUNG DAVON
COMPOSITION D'AGENT ÉDULCORANT, SON PROCÉDÉ DE PRÉPARATION ET SON UTILISATION

(30) Priority: 16.03.2022 CN 202210260674
(43) Date of publication of application: 25.09.2024
(73) Proprietor: Guilin Layn Natural Ingredients Corp., Guilin, Guangxi 541199 (CN)
(72) Inventor: WEI, Fengrui, Guilin, Guangxi 541199 (CN); ZHANG, Xueyu, Guilin, Guangxi 541199 (CN); CHEN, Xiaoying, Guilin, Guangxi 541199 (CN); HUANG, Jingxian, Guilin, Guangxi 541199 (CN); SONG, Yunfei, Guilin, Guangxi 541199 (CN)
(74) Representative: Sun, Yiming
(86) International application number: PCT/CN2022/118294
(87) International publication number: WO 2023/173705

(56) References cited:
- WO-A1-2018/040461
- CN-A- 101 376 901
- CN-A- 102 356 865
- CN-A- 106 962 882
- CN-A- 107 033 209
- CN-A- 108 740 930
- CN-A- 110 521 993
- CN-A- 111 296 708
- CN-B- 115 804 429
- JP-A- 2016 158 514
- JP-A- H08 173 109
- GONÇALO N. MARTINS ET AL: "Technological Aspects of the Production of Fructo and Galacto-Oligosaccharides. Enzymatic Synthesis and Hydrolysis", FRONTIERS IN NUTRITION, vol. 6, 31 May 2019 (2019-05-31), XP055721992, DOI: 10.3389/fnut.2019.00078

## Description

### Technical Field

The invention relates to a preparation method of a sweetened composition.

### Background Art

As one of the three major nutrients, sugar is the main energy source for human life activities. About 70% of the energy comes from sugar, which can also optimize the texture of the product and give the product a pleasant sweetness, and is a crucial factor affecting the taste. With the in-depth research on sugar, scientists have found that excessive intake of sugar has huge potential hazards: 1) Sugar rapidly increases the blood sugar content in the blood, and a long-term hyperglycemic state predisposes people to diabetes, resulting in electrolyte disturbance, impaired renal function, vasculopathy, neuropathy and other acute and chronic complications; 2) Sugar is high in calories and easily absorbed and transformed by the body, excess calories accumulate in the body, easily causing obesity and overweight, and resulting in increase in risks of cardiovascular and cerebrovascular diseases and some cancers; 3) Sugar can stimulate the proliferation of cariogenic bacteria in the mouth to form dental plaque, and constantly release corrosive substances to cause dental caries. To meet the health demand for sugar reduction, researchers usually use synthetic or natural sugar substitutes to reduce added sugar in sweetened situations. Momordica grosvenori, which belongs to natural fruits and has a long history of consumption, has been found to be an ideal and safe natural sugar substitute.

Momordica grosvenori is the fruit of the climbing herbaceous vine Siraitia grosvenorii [(Swingle) C. Jeffrey ex A. M. Lu et Z. Y. Zhang]. It is commonly found in Guangxi Province, Guizhou Province, Guangdong Province, Jiangxi Province and Southern Hunan. The main producing area is Guilin, Guangxi Province. Like various fruits and vegetables, fresh Momordica grosvenori has a certain fluctuation in composition due to different levels of maturity, roughly containing 78% water, 10% fiber, 8% sugar, 4% protein and other plant ingredients. Momordica grosvenori is characterized in that it contains no less than 20 tetracyclic triterpenoid glucosides, some of which have high sweetness intensity, about 100-500 times that of sucrose, and the most content is mogroside V, accounting for about 0.4% of the fresh fruit. In 2016, China introduced the national food safety standard GB1886.77-2016, which specifies the technical standards to be followed for mogroside in the Momordica grosvenori extract as a food additive. Mogroside is a powder product obtained by selective process steps such as water extraction, adsorption, decolorization, pesticide residue removal, concentration and drying.

Patent No. 201410190403.7 discloses an extraction method of mogroside V: mogroside products with different purities are obtained by extraction, multi-column adsorption, ion exchange resin refining, concentration, drying and other processes. The patent and its similar process based on adsorbent resin enrichment and ion exchange resin refining have low production costs, easily enabling large-scale production to be achieved, and being the traditional common production method in the industry. This process has the following technical defects: 1) The flavor, including amino acids, or its precursors are not completely removed, it is easy to produce an impure flavor during processing and storage, and the product is not stable in most solvent systems with high alcohol content; 2) More than 80% of protein, sugar and other nutrients are lost in the process, and resources are seriously wasted; 3) Ethanol and other organic solvents are used, which are flammable and explosive, and solvent residues exist in the product; 4) Due to the weak polarity and small powder particles, the product is slowly dissolved in water, increasing the application difficulty; 5) There are defects in sweetness: compared with traditional edible sugar, the obtained product has delayed sweetness, and the sweetness lasts longer in the mouth. Researchers in the field usually combine the product with sugar alcohol low-sweetness substances, so that the sweetness of the product is more similar to sucrose, but sugar alcohols have physiological tolerance and other problems, and more solutions are required to improve the sweetness.

Patent No. 201610387839.4 discloses a method for extracting high-purity mogroside V from Momordica grosvenori, including the following step: obtain the mogroside product by extraction, centrifugation, enzymolysis, ultrafiltration, nanofiltration, decolorization, microwave drying, crushing and other steps. In this process, more than 60% of the saccharides in the soluble components of Momordica grosvenori are lost through the membrane and cannot be used simultaneously, which is similar to the traditional resin process and results in waste of valuable Momordica grosvenori resources.

Concentrated juice products are usually obtained by selective steps such as squeezing, water extraction, clarification, cation- anion exchange and concentration. The products have purer taste than mogroside, and the resource utilization rate of Momordica grosvenori is improved by retaining the fruit saccharides with high calorie and high GI value. Patent No. 200810097509.7 discloses a decolorized Momordica grosvenori juice preparation method and a juice prepared by the method, which includes the following steps: select and pretreat materials, pass through the cation column, decolorize, acidify for the first time, concentrate, and acidify for the second time. The decolorized Momordica grosvenori juice is obtained by the combined process of cation column + anion column + cation column. Such products contain high carbohydrates and low mogroside. Among them, the sum of fructose, glucose and sucrose accounts for more than 80% of the dry matter weight, the content of mogroside V is about 2-4%. The negative result is that the overall sweetness of the juice is not high, only about 10-15 times that of sucrose. A relatively large amount of addition is required for sweetening, which limits the application scenarios, and the standards of zero sugar and zero calorie cannot be met during use in yogurt or other high-sweetness formulas. Meanwhile, the proportion of three saccharides has relatively large fluctuations in the natural state. When the glucose content is too high, the glucose easily forms white crystals that precipitates at temperature close to 0 degree C in winter in cold regions, affecting the stability of the juice state.

WO2018040461A1 discloses a natural plant product compound sweetener, prepared from momordica grosvenori dietary fiber, mogroside V, fructo-oligosaccharide, erythritol, palatinitol and malic acid by solution in water, filtration, vacuum concentration and spray drying.

JP2016158514A provides a momordicae fruit sweetening component-containing composition and others that contains mogrosides commonly known as a sweetening component of momordicae fruit, exhibits a good sweetness quality, and also has an excellent function such as an antioxidative property.

JPH08173109A provides a liquid food raw material having the original taste and flavor of a farm product and utilizable as a drink having harmonized sourness and sweetness and for the preparation of a seasoning having unique taste and flavor.

CN102356865A discloses a sweetener and a preparation method thereof. The sweetener contains, based on dry weight, 20-98% of total mogroside from grosvenor momordica fruit, wherein mogroside V accounts for 5-80%. When the sweetener completely dissolves in water and has a same sweetness as a 5% (w/w) cane sugar aqueous solution under a same condition, the sweetener aqueous solution has a 210nm absorbance of 1.2-1.4. The preparation method of the sweetener comprises steps of: adding water and pectase into grosvenor momordica fruit for extraction, extracting injecting liquid, loading on a macroporous resin column and collecting an ethanol eluate; flowing an eluate through a neutral alumina column, collecting an effluent, recovering the ethanol, concentrating and drying. Compared with a prior art, the sweetener in the above limited scope is in light yellow or white, and has no traditional Chinese medicine taste or bitter taste of grosvenor momordica fruit; and the preparation method is simple, at low costs and easy for industrialized production.

CN101376901A relates to a preparation method of high-purity low fructosan, which belongs to the technical field of purifying low fructosan by organically integrating barrier separation and enzyme reaction. In the invention, common low fructosan serves as the raw material; enzyme reaction and barrier separation are conducted in a barrier and enzyme reactor, the content of monosaccharide and sucrose is reduced to obtain the high-purity low fructosan. The content of the low fructosan obtained through the method is above 95 percent; the method has the advantages of convenient operation, stable quality, safety and reliability, and is applicable to industrialized production.

To sum up, the existing highly processed products from Momordica grosvenori are difficult to meet the comprehensive market requirements for pure taste, low sugar and low calorie, faster dissolution, and sweet taste closer to sugar.

### Summary of the Invention

Aiming at the defects of the prior art, the invention aims to provide a sweetened composition, the enzymatic conversion treatment is performed on the Momordica grosvenori extract by biological enzyme coupled membrane separation technology, sucrose contained in Momordica grosvenori is converted into oligofructose under the action of fructosyltransferase (EC 2.4.1.9), sucrase and glucose isomerase, low-sweetness glucose commonly existing in the Momordica grosvenori is also converted into high-sweetness fructose, so that the prepared sweetened composition product not only has low sugar and low calorie, but also has purer taste and thicker flavor, is closer to edible sugar, and improves the defects of insufficient sweetness and delayed sweetness of mogroside V.

To achieve the above-mentioned object, the invention is set out in the appended claims.

Not according to the invention is a sweetened composition comprising at least 0-99% mogroside V and 0-99% fructooligosaccharide. Preferably, the sweetened composition comprises at least 0-55% mogroside V and 0-40% fructooligosaccharide. Further preferably, the sweetened composition comprises at least 0-35% mogroside V and 0-20% fructooligosaccharide. The sweetened composition has the characteristics of low sugar, low calorie, high sweetness, purer taste and thicker flavor, contains fructooligosaccharide prebiotics, which is beneficial to human health and improves the defect of delayed sweetness of mogroside V.

In the invention, the fructooligosaccharide includes at least one or more of fructooligosaccharide GF1, fructooligosaccharide GF2 and fructooligosaccharide GF3.

In the invention, the biological enzyme is fructosyltransferase or microorganism containing the fructosyltransferase.

Further, the added content of the biological enzyme is measured by the added content of the fructosyltransferase, which is 0.1-0.5% of the total content of soluble solid matters in the Momordica grosvenori extract. In the invention, the enzymatic conversion treatment is performed on the Momordica grosvenori extract by biological enzyme coupled membrane separation technology, fructosyltransferase transfers a fructose F in one sucrose GF molecule in the Momordica grosvenori extract to another sucrose molecule to form a fructooligosaccharide GF1 and generate one molecule of glucose, and continues to take GF1 as a substrate to form a fructooligosaccharide GF2 and generate one molecule of glucose, and form a fructooligosaccharide GF3 and glucose in the same way. The glucose product generated by enzymatic conversion is separated from a reaction system under the action of a semi-permeable membrane in an enzyme membrane bioreactor, thus being conducive to the forward progress of the enzymatic conversion reaction and the formation of higher concentration fructooligosaccharide. As the conversion proceeds, the sucrose is converted into the fructooligosaccharide and concentrated continuously on the membrane interception side, and monosaccharides such as glucose and fructose as well as water are separated from the membrane permeation side. The membrane used in the enzymatic membrane bioreactor is a commercially available semi-permeable membrane that allows glucose and water to permeate, and the semi-permeable membrane can be a bag type, a roll type, a tube type or any other form.

In the invention, the biological enzyme can be free or carrier type, the carrier of the carrier biological enzyme includes but is not limited to one or more of alumina, diatomaceous earth, resin, cellulose, gel, ceramic membrane and organic membrane.

In the invention, the microorganisms containing fructosyltransferase include but are not limited to aspergillus niger, aspergillus oryzae, aureobasidium pullulans, fusarium sp., arthrobacter sp., bacillus subtilis and saccharomyces.

In the invention, the added content of the sucrase is 0.01-0.8% of the total content of the soluble solid matters in the juice aqueous solution, the added content of the glucose isomerase is 0.1-2.5% of the total content of the soluble solid matters in the juice aqueous solution, and the reaction is performed for 1-3 hours after the sucrase and the glucose isomerase are added. In the invention, the juice aqueous solution on the membrane permeation side is taken as the raw material, in which sucrase and glucose isomerase are added, the sucrose in the juice aqueous solution is decomposed into monosaccharides under the action of the sucrase, and the glucoses in the monosaccharide and the juice aqueous solution are converted into fructose under the action of the glucose isomerase. After enzymatic decomposition and isomerization, the sucrose in the juice aqueous solution is completely decomposed, about 0.6X sweetness glucose is converted into 1.5X sweetness fructose, and the fructose conversion liquid with increased sweetness is obtained, namely, the calorie and sugar content of the juice with unit sweetness are reduced, so that the prepared sweetened composition is low in sugar and calorie. Meanwhile, the glucose content in the isomerized fructose conversion liquid is reduced, which is conducive to maintaining the stability of the sweetened composition at low temperature. Further, the sucrase or glucose isomerase in the invention can be a live bacterium containing sucrase or glucose isomerase, a bacterial cell, or a commercial enzyme-containing product purified therefrom.

In the invention, the Momordica grosvenori extract is obtained by extracting Momordica grosvenori with water and then filtering to remove impurities; specifically, the added amount of water is subject to immersion of the Momordica grosvenori; preferably, the mass ratio of the water to the Momordica grosvenori is 2-5:1, the extraction temperature is 80-90 degrees C, the extraction time is 20- 40 minutes, and the number of extraction times is 1-3; preferably, the number of extraction times is 2-3, and the extraction time is 20-30 minutes; the pore size of a filter membrane used in filtration is less than or equal to 1 µm. In the invention, the Momordica grosvenori can be extracted by reflux extraction or countercurrent extraction. A filtration membrane with a pore size less than or equal to 1 µm is used to filter visible wood fibers, debris or colloidal substances in the extract, including but not limited to a ceramic membrane.

In the invention, after-ripening treatment is required to be conducted on the Momordica grosvenori for 1-10 days before the Momordica grosvenori extract is obtained. Specifically, the Momordica grosvenori fruit is picked and placed in a special wooden cabinet, which is placed in a fruit shed with good insect prevention, rain shelter, ventilation and lighting for after-ripening treatment, and a series of composition and decomposition processes of the picked fruit are utilized to reduce the acid and bitter components of the fruit, improve the conversion of other low-sugar base glycosides to mogroside V and increase the content of mogroside V in the fruit. In the invention, Momordica grosvenori fruits of different maturities can be used, including three increasing grades of green fruit, greenish yellow fruit and yellow fruit. The criteria of the green fruit are that the surface color of the pericarp is mostly cyan, and the flesh is hard and low in maturity; the yellow fruit refers to the ripe fruit whose pericarp surface is mostly yellow and whose flesh has been softened; the greenish yellow fruit is between green fruit and yellow fruit. Different after-ripening times are selected according to the characteristics of fruits with different ripeness. Specifically, the after-ripening treatment time of the green fruit is 7-10 days, the after-ripening treatment time of greenish yellow fruit is 5-8 days, and the after-ripening treatment time of the yellow fruit is 1-5 days.

In the invention, the Momordica grosvenori extract can be purified with resin before the Momordica grosvenori extract is transferred to the enzyme membrane bioreactor, and the resin comprises one or more of adsorbent resin, anion resin and cation resin. The resin is mainly used for decolorization and adsorption of organic matters, can effectively remove impurities in the Momordica grosvenori extract, has main influence on the appearance color of the product and the content of amino acids and mogroside V, and has a certain positive effect on the stability of the product.

In the invention, a low temperature concentrator can be used for concentration, and comprises a rotary concentrator, a single-effect falling film concentrator, a multi-effect falling film concentrator, a multi-effect rising film concentrator or other low temperature concentration plants. Concentration is performed to BRIX more than 20% at the concentration temperature of 60-80 degrees C, and; preferably, concentration is performed to BRIX of 60-70% at the concentration temperature of 70 degrees C.

In the invention, sterilization can be done by autoclaved sterilization, heat sterilization, microwave sterilization, high temperature instantaneous sterilization or membrane filtration sterilization. High temperature instantaneous sterilization and membrane filtration sterilization are preferred to obtain a sweetened composition concentrate.

In the invention, the enzymatic conversion liquid or juice aqueous solution is concentrated, sterilized and then dried to obtain the powdery sweetened composition, and the drying treatment is belt drying or spray drying. One of the differences between the powdery sweetened composition and the traditional mogroside sweetener powder is that the powdery sweetened composition has more rapid water absorption capability and dissolves more quickly, and is more convenient for application where dissolution is required. Specifically, belt drying or spray drying is used, the belt drying is performed at the process conditions of 70-90 degrees C in sections 1-4, 20-28 degrees C in section 5, material temperature of 40-50 degrees C, fabric angle of 5-8 degrees and pressure of 2000-3000 Pa; the spray drying is performed at the process conditions of inlet air temperature of 130-170 degrees C, outlet air temperature of 75-85 degrees C and negative pressure of 110-140 Pa in the tower.

Not according to the invention is a use of the sweetened composition in the preparation of food, beverages, dietary supplements, pharmaceuticals and pet food. The sweetened composition prepared by the invention can be diluted with water into a Momordica grosvenori drink and consumed directly; the sweetened composition can be added separately for sweetening to make the product sweeter; the sweetened composition can substitute or reduce common natural sweet substances such as sucrose, high fructose corn syrup, honey, stevioside, neotame or sucralose as well as artificial sweeteners to make the product healthier; the sweetened composition can modify the taste, and mix with various natural and synthetic sweet substances to cover up the bitter, astringent, sour and other bad taste of the product; the sweetened composition can modify and make the flavor more soft and pleasant; the sweetened composition can moisturize and keep the product moist.

The invention has the following beneficial effects:
(1) The sweetened composition prepared by the invention contains mogroside V and soluble dietary fiber (prebiotics), and the prebiotics includes at least one or more of fructooligosaccharide GF1, fructooligosaccharide GF2 and fructooligosaccharide GF3, and can n promote the growth and propagation of probiotics in human body and is conducive to improving human health; in addition, the sweetened composition prepared by the invention can improve the defects of slow sweetening and delayed sweetness of mogroside V, and make the product taste purer and thicker to be closer to edible sugar.
(2) The sweetened composition prepared by the invention is more stable in the organic solvent system of propylene glycol, glycerol and other alcohols, has low sugar, low calorie and higher dissolution speed, and has wider application value than the existing Momordica grosvenori extracts.
(3) In the preparation method of the sweetened composition provided by the invention, the enzymatic conversion treatment is performed on the Momordica grosvenori extract by biological enzyme coupled membrane separation technology, the nutrient carbohydrate resource (sucrose) which is common in the Momordica grosvenori is converted to healthy prebiotics (fructooligosaccharide) under the action of fructosyltransferase (EC 2.4.1.9), sucrase and glucose isomerase existing in the biological enzyme and produced by microbial fermentation, and the low-sweet glucose is converted to the high-sweet fructose. By the recombination of components, the value chain of Momordica grosvenori is extended, the sweetness of the product is improved, and the defects of slow sweetening and delayed sweetness of mogroside V are filled, and the problem of insufficient sweetness of the product is improved.
(4) The preparation method of the sweetened composition provided by the invention also includes the following steps: perform after-ripening treatment on the Momordica grosvenori fruits, utilize a series of composition and decomposition processes of the picked fruit to reduce the acid and bitter components, improve the conversion of other low-sugar base glycosides to mogroside V and increase the content of mogroside V in the fruit. The preparation method makes the selection of raw materials not affected by the ripeness of the fruit while improving the recovery rate.
(5) In the preparation method of the sweetened composition provided by the invention, the glucose product generated by the enzymatic conversion is separated from the reaction system under the action of the semi-permeable membrane, which is conducive to the continuous forward bioconversion and the formation of a higher concentration fructooligosaccharide on the membrane interception side.
(6) The invention relies on biotechnology and membrane technology, does not involve organic solvents with explosion and residual safety hazards, and is more friendly to people and the natural environment.

### Brief Description of the Drawings

FIG. 1 is a sweetness curve in the embodiment 2 of the invention; in the figure, curve 1 is the sweetness curve for sucrose, curve 2 is the sweetness curve for the sweetened composition product of the embodiment 2, curve 3 is the sweetness curve for the traditional mogroside MV30.
FIG. 2 is a structure diagram for the enzyme membrane bioreactor of the invention; in the figure, 1 is the fermentation tank, and 2 is the semi-permeable membrane.

### Detailed Description of the Invention

The invention is further described below in combination with the embodiments.

### Term Interpretation

For the interpretation of the technical terms used in the invention, BRIX is equivalent to the mass fraction of soluble solid matters, for example, BRIX of juice solution is 30%, indicating that the mass fraction of the dry matters in juice is approximately 30%. Sweetness refers to the intensity of sweetness, with reference of sucrose aqueous solution with mass concentration of 5%. The standards for zero sugar and zero calorie are derived from Chinese national food safety standard GB28050-2011 General Rules for Nutrition Labeling of Prepackaged Food. Zero sugar means that the content of carbohydrate in the food, namely sugar, is less than 0.5 g/100g solid or 100ml liquid. Zero calorie means that the total energy in the food is less than or equal to 17 KJ/100g solid or 100ml liquid. BV is short for column bed volume.

### Embodiment 1

(1) After-ripening treatment: Manually sort out 1.4 tons of yellow fruits from the newly picked Momordica grosvenori fruits, place the screened fruits in special wooden frames to prevent from being piled up and squashed, transfer to a fruit shed with good insect prevention, rain shelter, ventilation and lighting, and store for 2 days.
(2) Obtain juice: Add the after-ripened Momordica grosvenori fruits in Step (1) to 2.8 tons of potable water , extract for 20 minutes at 82 degrees C, collect the Momordica grosvenori extract, and repeat extraction. Combine the Momordica grosvenori extracts to obtain 6,600 L of Momordica grosvenori extract containing juice in total, with BRIX of 2.5% and pH of 5.6.
(3) Pretreatment: Filter the Momordica grosvenori extract in Step (2) through a plate and frame filter with a filter cloth pore size of 0.45 µm, back-wash by 400 L to remove visible wood fiber bits, colloid and other macromolecular substances contained in the extract, and obtain about 7,000 L of clarified Momordica grosvenori extract.
(4) Enzymatic conversion: Perform enzymatic conversion on the clarified Momordica grosvenori extract in Step (3) by biological enzyme coupled membrane separation technology to convert nutritional saccharides into fructooligosaccharide. Transfer the clarified Momordica grosvenori extract into an enzyme membrane bioreactor, which comprises a fermentation tank 1 and a semi-permeable membrane 2, transfer the Momordica grosvenori extract to the fermentation tank 1 for enzymatic conversion, and then separate through the semi-permeable membrane 2. Add 300 ml of fructosyltransferase to the enzyme membrane bioreactor, mix, and set the membrane feeding pressure of the membrane equipment to 0.2-0.6 Mpa, the membrane separation flux to 200-800 L/H, the enzymatic conversion temperature to 42 degrees C and pH to 5.5. Continuously separate and concentrate materials while converting, and the conversion is completed when the volume of membrane permeate reaches about 6,600 L. Back-wash by 200 L, collect the concentrate on the membrane interception side to obtain 600 L of enzymatic conversion liquid with BRIX of 5.5%, and collect about 6,800 L of juice aqueous solution on the membrane permeation side.
(5) Decomposition and isomerization of sugar: In about 6,800L of membrane permeate in Step (4), set the temperature to 53 degrees C, uniformly add 120 ml of sucrase and 500 ml of glucose isomerase, thermally insulate for 3 hours, and obtain the fructose conversion liquid.
(6) Recombination: 600 L of enzymatic conversion liquid in Step (4) is not added to the fructose conversion liquid in Step (5), namely, the proportion of the enzymatic conversion liquid is 100%.
(7) Concentration: Transfer the enzymatic conversion liquid to four small rotary evaporators with an evaporation capacity of 50 L/H, concentrate to BRIX of 70% at temperature of 70 degrees C and vacuum of 0.1 Mpa, and obtain the juice concentrate.
(8) Sterilization: Transfer the juice concentrate in Step (7) to high temperature instantaneous sterilization equipment, set the temperature to 125 degrees C, and obtain the sterilized juice concentrate.
(9) Obtain the sweetened composition: Transfer the sterilized juice concentrate to belt drying equipment for drying, and reduce the moisture to be less than 6% to obtain the finished product. Set the belt drying temperature to 80 degrees C in the sections 1-4 and 25 degrees C in the section 5, the material temperature to 45 degrees C, the fabric angle to 5 degrees and pressure to 2200 Pa, and obtain 33 kg of dried powdery sweetened composition.

Through the detection of the content of components in the sweetened composition, the content of mogroside V is 15.6%, the content of fructooligosaccharide is 14.7%, the intensity of sweetness is 70-80 times that of sucrose, the content of carbohydrate is 76.5 g/100g, and the energy is 1,361 KJ/100g.

### 1) Taste test

The mogroside powder product prepared by the traditional process was taken as the control group to conduct a random triple blind taste test. Triple blind refers to blind to taste liquid preparer, tasters and result statistician. The control group product was from Guilin Layn Natural Ingredients Co., Ltd., with lot number of LHG220116-03. The content of mogroside V was 15.2%. The main process steps were extraction, clarification, resin adsorption chromatography, ethanol analysis, pesticide residue removal and spray drying.

The sweetened composition prepared in the embodiment and the control group product were respectively prepared into solutions with the content of mogroside V of 200 PPM and held in opaque containers, in which the sweetened composition solution was contained in one cup, and the control group solution was contained in the other cup. 20 testers randomly tasted and selected a product with higher purity. The statistical results showed that all testers believed that the flavor of the embodiment was purer, and almost all testers believed that the sweet aftertaste of the embodiment was shorter.

| Name | Content of mogroside V of product | Content of mogroside V of taste test liquid | Number of testers who think the flavor is purer | Number of testers who think slow sweetening and short sweetness |
|---|---|---|---|---|
| Embodiment 1 | 15.6% | 200 PPM | 20 | 18 |
| Control group | 15.2% | 200 PPM | 0 | 2 |

### 2) Sugar and calories introduced by product sweetening

LHXT-N01 LHZ-210112-01 decolorized Momordica grosvenori juice concentrate from Guilin Layn Natural Ingredients Co., Ltd. was taken as the control group; the control group product and the sweetened composition prepared in the embodiment were prepared into simple pure water beverages by the sweetness of 30 g per 100 ml of water, and the sugar and calorie introduced were calculate when the sweetness was applied, as shown in the table below.

| Name | Sugar content | Calories | Sweetness | Added content | Increased sugar | Increased calories |
|---|---|---|---|---|---|---|
| Unit | g/100g | KJ/100ml | Times | % | g/100ml | KJ/100ml |
| Embodiment 1 | 76.5 | 1361 | 75 | 0.4 | 0.3 | 5.4 |
| Control group | 61.7 | 1136 | 11 | 2.7 | 1.7 | 30.7 |

The results showed that both were prepared according to the sweetness standard of 30 g of sucrose per 100 ml of water, the sweetened composition in the embodiment introduced 0.3 g/100ml sugar and 5.4 KJ/100ml calorie, meeting the standard requirements of zero sugar and zero calorie, and the control group introduced 1.7 g/100ml sugar and 30.7 KJ/100ml calorie, not meeting the requirements. It can be seen that the sweetened composition of the invention added to the beverage products as a sweetener not only greatly increases the sweetness, but also meets the standard requirements of zero sugar and zero calorie.

### 3) Applications

A. Juice beverage: Put 0.1 part of the sweetened composition prepared in the embodiment into a commercially available commercial juice beverage dispenser, add 100 parts of sterile water, stir evenly, and directly dilute to prepare a drinkable fresh and sweet Momordica grosvenori beverage.
B. Green tea: Take 0.007 part of the sweetened composition prepared in the embodiment and 100 parts of freshly brewed green tea, and obtain a significantly sweetened, less bitter green tea beverage with significant aftertaste.
C. Reduced sugar coffee: Take 0.04 part of the sweetened composition prepared in the embodiment, 100 parts of freshly brewed coffee and 0.25 part of decolorized Momordica grosvenori juice, and obtain the coffee drink with reduced sugar by approximately 85% than the traditional added sugar and reduced bitterness without change of original flavor of the coffee.

### Embodiment 2

(1) After-ripening treatment: Manually sort out 20 tons of greenish yellow fruits from the newly picked Momordica grosvenori fruits, place the fruits in special wooden frames and transfer to a fruit shed with good insect prevention, rain shelter, ventilation and lighting, and store for 6 days.
(2) Obtain juice: Feed the ripened greenish yellow fruits into countercurrent extraction equipment, keep the fruit feeding speed at about 6 tons/H and the water adding speed at about 20 tons/H, set the temperature to 85 degrees C, and obtain 70 tons of Momordica grosvenori extract.
(3) Pretreatment: Clarify the Momordica grosvenori extract by 1 µm ceramic microfiltration membrane, and transfer the clarified Momordica grosvenori extract to a decolorization column composed of a cation resin column D001*7 (provided by SUNRESIN) and an anion resin column LX-T5 (provided by SUNRESIN) which are connected in series for decolorization at a flow rate of 3.5 BV/H. Set the total liquid treatment amount of the resin to 20 BV and the flow rate to 1 BV/H. After the feeding is completed, back-wash by 5 BV, and collect the decolorized Momordica grosvenori extract.
(4) Enzymatic conversion: Set the decolorized Momordica grosvenori extract to 48 degrees C by a heat exchanger, adjust citric acid pH to 4.5-5.8, continuously transfer to the enzyme membrane bioreactor at a flow rate of 12,000 L/H and a membrane pressure of 0.5-1.5 Mpa. Meanwhile, prepare 7 L of fructosyltransferase liquid, and continuously and evenly add the fructosyltransferase liquid to the Momordica grosvenori extract by a peristaltic pump. Set the same flux of the semi-permeable membrane in the enzyme membrane bioreactor as the flow rate of the Momordica grosvenori extract of 12,000 L/H, and continuously feed and convert the Momordica grosvenori extract. When the material in the reactor is concentrated to about 15 tons, add 5 tons of water and continue the cycle for 3 hours to complete the enzymatic conversion, obtain 9 tons of enzymatic conversion liquid on the membrane interception side, and obtain 80 tons of juice aqueous solution on the membrane permeation side.
(5) Decomposition and isomerization of sugar: Continuously and evenly add 1 L of sucrase and 20 L of glucose isomerase to the juice aqueous solution in Step (4) by the peristaltic pump, thermally insulated for 3 hours, and obtain about 80 tons of fructose conversion liquid with increased sweetness.
(6) Recombination: Evenly mix about 9 tons of 100% enzymatic conversion liquid in Step (4) with 10% sweetened fructose conversion liquid in Step (5), and obtain a recombined mixture.
(7) Concentration: Transfer the recombined mixture in Step (6) to an MVR (mechanical vapor recompression) evaporator, set the concentration temperature to 62 degrees C, concentrate to about BRIX of 63.5% to complete the concentration, and the total amount of concentrate is about 600 L.
(8) Sterilization: Transfer the juice concentrate in Step (7) to high temperature instantaneous sterilization equipment, set the temperature to 120 degrees C, and obtain the sterilized juice concentrate.
(9) Obtain the sweetened composition: Transfer the sterilized juice concentrate to spray drying equipment, set the inlet air temperature to 150 degrees C, the outlet air temperature to 80 degrees C and the negative pressure in the tower to 120 Pa, and obtain about 412 kg of dry powdery sweetened composition.

Through the detection of the sweetened composition product, the content of mogroside V is 18.7%, the content of fructooligosaccharide is 4.5%, and the intensity of sweetness is 90-100 times that of sucrose.

### 1) Taste test

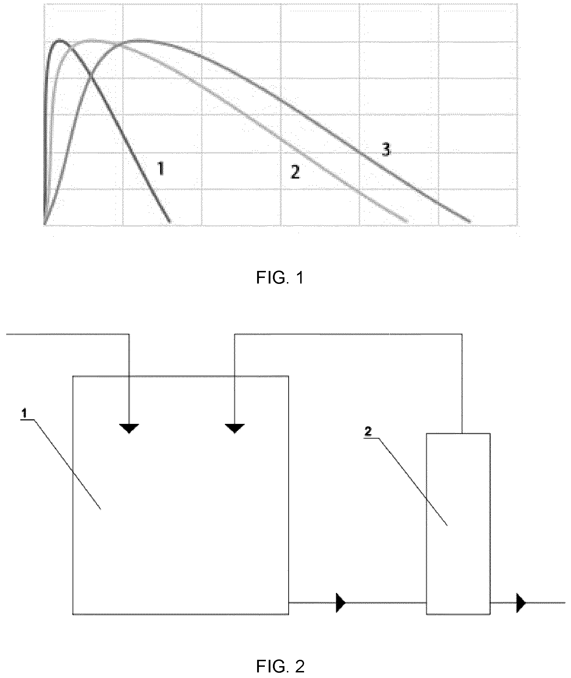

The sucrose, the sweetened composition product prepared in the embodiment and the traditional mogroside product MV30 (selected from Guilin Layn Natural Ingredients Co., Ltd.) were prepared into 5% sucrose sweetness aqueous solutions. Three samples were evaluated by four sensory taste testers, the felt sweetness changes and the corresponding time were recorded with a stopwatch, and the time-sweetness curve was drawn, as shown in FIG. 1.

The results showed that the sweet taste of the sweetened composition in the embodiment reached the peak time earlier, the sweet taste duration was slightly shorter and the sweetness curve was closer to that of the sucrose compared with the traditional mogroside product MV30.

2) The radar chart for different sensory indexes of the three products is drawn as shown in FIG. 2. The evaluation standards and results are shown in the following table:
Evaluation standards:

| Degree | None | Extremely weak | Very weak | Weak | General | Strong | Very strong | Extremely strong |
|---|---|---|---|---|---|---|---|---|
| Score | 0 | 1 | 2 | 3 | 4 | 5 | 6 | 7 |

Elevation results of indexes:

| | MV30 | Sweetened composition in the embodiment | Sucrose |
|---|---|---|---|
| Smell | 7 | 2 | 2 |
| Purity | 1 | 6 | 6 |
| Richness | 5 | 4 | 6 |
| Sour | 0 | 0 | 0 |
| Bitterness | 1 | 0 | 0 |
| Preference | 2 | 5 | 5 |

The above evaluation results show that the sweetened composition of the invention is basically consistent with sucrose in purity, smell and preference in the above indexes, except that the richness is slightly worse than that of sucrose. The traditional mogroside product MV30 has poor purity, obvious special smell and slight bitter taste.

### 3) Applications

A. Fresh low-sugar soymilk: Take 0.05 part of the sweetened composition prepared in the embodiment, 100 parts of soymilk and 3 parts of Momordica grosvenori juice (without mogroside) to obtain about 7% sucrose sweetness soymilk product, but the actual sugar content of the product is only 3%, reducing about 60% of added sugar, and the taste is fresher and more natural, without the sweet taste of sugar.
B. Compound sweetener: Take 3 parts of the sweetened composition prepared in the embodiment, 1.7 parts of stevia, 1 part of sucralose, 0.3 part of neotame and 14 parts of resistant dextrin, mix, and obtain the sweetener similar to sucrose and having no lingering bitterness.
C. Toothpaste: Take 0.1 part of sweetened composition, 50 parts of mixed abradant, 20 parts of glycerin, 3 parts of sodium N-lauroylglutamate and 1 part of carboxymethyl cellulose, and add water to 100 parts.

### Embodiment 3

(1) After-ripening treatment: Manually sort out 100 kg of green fruits from the newly picked Momordica grosvenori fruits, place the fruits in special wooden frames and transfer to a fruit shed with good insect prevention, rain shelter, ventilation and lighting, and store for 10 days.
(2) Obtain juice: Add the after-ripened Momordica grosvenori fruits in Step (1) to 300 L of potable water , extract for 30 minutes at 90 degrees C, collect the Momordica grosvenori extract, and repeat extraction. Combine the Momordica grosvenori extracts to obtain 670 L of Momordica grosvenori extract, with BRIX of 1.7% and pH of 5.7.
(3) Pretreatment: Filter the Momordica grosvenori extract in Step (2) through a ceramic membrane with a pore size of 0.2 µm, and back-wash by 30 L to remove visible wood fiber bits, colloid and other macromolecular substances contained in the extract. Transfer the clarified Momordica grosvenori extract to a decolorization column composed of a cation resin column D001*7 (provided by SUNRESIN), an anion resin column LX-T5 (provided by SUNRESIN) and a cation resin column D001*7 (provided by SUNRESIN) which are connected in series for decolorization. The ratio of cation resin to anion resin to cation resin is 1:1:1. Set the liquid treatment amount of the resin to 35 BV and the feed flow rate to 1.5 BV/H. After the feeding is completed, back-wash by 5 BV, and obtain 780 L of pretreatment liquid with BRIX of 1.0%.
(4) Enzymatic conversion: Perform enzymatic conversion on the clarified Momordica grosvenori extract in Step (3) by biological enzyme coupled membrane separation technology to convert nutritional saccharides into prebiotics fructooligosaccharide. Transfer the clarified Momordica grosvenori extract into a storage tank of enzyme membrane bioreactor prototype equipment, add 22 ml of fructosyltransferase, mix, and set the membrane feeding pressure of the membrane equipment to 0.5-1.0 Mpa, the membrane separation flux to 70-90 L/H, the enzymatic conversion temperature to 48 degrees C and pH to 4.0. Continuously separate and concentrate materials while converting. Membrane permeate enters the sugar decomposition and isomerization process. The conversion is completed when the volume of permeate reaches about 660 L. Back-wash by 30 L, and collect the concentrate on the membrane interception side to obtain 150 L of enzymatic conversion liquid with BRIX of 1.1%; collect about 680 L of juice aqueous solution on the membrane permeation side.
(5) Decomposition and isomerization of sugar: Continuously and evenly add 10 ml of sucrase and 160 ml of glucose isomerase to the juice aqueous solution on the membrane permeation side in Step (4) by the peristaltic pump, thermally insulated for 2 hours, and obtain about 680 L of fructose conversion liquid with increased sweetness.
(6) Recombination: Evenly mix about 150 L of 100% enzymatic conversion liquid in Step (4) with about 30 L of 5% sweetened fructose conversion liquid in Step (5), and obtain a recombined mixture
(7) Concentration: Transfer the recombined mixture in Step (6) to a rotary evaporator with an evaporation capacity of 50 L/H, set the concentration temperature to 75 degrees C and the vacuum of 0.1 Mpa, concentrate to about BRIX of 65% to complete the concentration, and the total amount of concentrate is about 2.0 L.
(8) Sterilization: Transfer the juice concentrate in Step (7) to high temperature instantaneous sterilization equipment, set the temperature to 120 degrees C, and obtain the sterilized juice concentrate.
(9) Obtain the sweetened composition: Transfer the sterilized juice concentrate to belt drying equipment for drying, and reduce the moisture to be less than 6% to obtain the finished product. Set the belt drying temperature to 80 degrees C in the sections 1-4 and 25 degrees C in the section 5, the material temperature to 45 degrees C, the fabric angle to 5 degrees and pressure to 2,200 Pa, and obtain 1.5 kg of dried powdery sweetened composition.

Through the detection of the content of components in the sweetened composition, the content of mogroside V is 20%, the content of fructooligosaccharide is 8.2%, and the intensity of sweetness is 100 times that of sucrose.

### 1) Stability test of organic solvents

Respectively place 2.0 g of the sweetened composition in the embodiment and 2.0 g of traditional mogroside (with the content of mogroside V of 30±1%, from Guilin Layn Natural Ingredients Co., Ltd., with lot numbers of LHG210128-2, LHG210310-1 and LHG210516-1) in 250 ml glass sample bottles, add 10 ml of pure water and 88.0 g of analytical pure glycerol, stir until completely dissolved, and stand at room temperature for 7 days. Point the bottom of the sample bottles at a strong light source and observe the state of the solution in the bottles. Three parallel repetitions were set up for the experiment. The results showed that there were a large number of bulk precipitates in three traditional mogroside products, and the solutions were turbid. The sample solutions of the sweetened compositions in the three embodiments were clear without significant precipitation. Obviously, the sweetened composition of the embodiment has better organic solvent stability than the traditional mogroside product.

### 2) Room temperature solubility test

Respectively weigh and place 10.0 g of traditional mogroside (from Guilin Layn Natural Ingredients Co., Ltd., with lot numbers of LHG210415-01 and LHG210303-02) and 10.0 g of sweetened composition in the embodiment in 250 ml conical bottles, add 90 ml of pure water, stir manually in the same direction until completely dissolved, and record the time. The results showed that the complete dissolution time of the sweetened composition in the embodiment was 38 seconds, the complete dissolution time of the traditional mogroside product LHG210303-02 was 7 minutes, and the complete dissolution time of the traditional mogroside product LHG210415-01 was 6 minutes. Obviously, the sweetened composition of the embodiment dissolves faster and is easy to use.

### 3) Applications

A. Pudding chocolate milk tea: Take 0.1 part of the sweetened composition prepared in the embodiment, 20 parts of pudding, 5 parts of chocolate powder, 4 parts of whole milk powder and 2 parts of honey, add hot water to 100 parts, disperse and mix, and add a small amount of ice.
B. E-liquid essence: Take 20 parts of the sweetened composition prepared in the embodiment, 40 parts of propylene glycol and 40 parts of glycerin, dissolve and evenly mix.
C. Koi diet: Take 0.2 part of the sweetened composition prepared in the embodiment, 40 parts of extruded maize meal, 30 parts of dried peanut powder, 10 parts of snail meat powder, 10 parts of fish meal and 10 parts of spirulina, mix evenly, and granulate.

### Embodiment 4

(1) After-ripening treatment: Manually sort out 50 kg of greenish yellow fruits from the newly picked Momordica grosvenori fruits, place the screened fruits in special wooden frames to prevent from being piled up and squashed, transfer to a fruit shed with good insect prevention, rain shelter, ventilation and lighting, and store for 1 day.
(2) Obtain juice: Add the after-ripened Momordica grosvenori fruits in Step (1) to 150 L of potable water , extract for 20 minutes at 80 degrees C, collect the Momordica grosvenori extract, and repeat extraction. Combine the Momordica grosvenori extracts to obtain 310 L of Momordica grosvenori extract containing juice in total, with BRIX of 2.0% and pH of 5.1.
(3) Pretreatment: Clarify the Momordica grosvenori extract by 0.2 µm ceramic microfiltration membrane, and transfer the clarified Momordica grosvenori extract to a decolorization column composed of a cation resin column D001*7 (provided by SUNRESIN), an anion resin column LX-T5 (provided by SUNRESIN) and cation resin column D001*7 (provided by SUNRESIN) which are connected in series for decolorization at a flow rate of 2-4 BV/H. Set the total liquid treatment amount of the resin to 20 BV and the flow rate to 1-2 BV/H. After the feeding is completed, back-wash by 5 BV, and collect the decolorized Momordica grosvenori extract.
(4) Enzymatic conversion: Dissolve 20 ml of fructosyltransferase in 50 L of pure water, transfer to a small enzyme membrane bioreactor for continuous circulation for 20 minutes, fix fructosyltransferase on a spiral-wound membrane carrier to complete enzyme immobilization. Transfer the decolorized Momordica grosvenori extract to the enzyme membrane bioreactor. Set the membrane feeding pressure of the membrane equipment to 0.1-0.5 Mpa, the membrane separation flux to 20-50 L/H, the enzymatic conversion temperature to 45 degrees C and pH to 5.2. Continuously separate and concentrate materials while converting, and the conversion is completed when the volume of membrane permeate reaches about 350 L. Back-wash by 10 L, collect the concentrate on the membrane interception side to obtain 50 L of enzymatic conversion liquid.
(5) Decomposition and isomerization of sugar: In about 350L of membrane permeate in Step (4), set the temperature to 53 degrees C, uniformly add 5 ml of sucrase and 100 ml of glucose isomerase, thermally insulate for 3 hours, and obtain the fructose conversion liquid.
(6) Recombination: 50 L of enzymatic conversion liquid in Step (4) is not added to the fructose conversion liquid in Step (5), namely, the proportion of the enzymatic conversion liquid is 100%.
(7) Concentration: Transfer the enzymatic conversion liquid to a small rotary evaporator with an evaporation capacity of 20 L/H, concentrate to BRIX of 55% at temperature of 72 degrees C and vacuum of 0.08 to 0.1 Mpa, and obtain the juice concentrate.
(8) Sterilization: Transfer the juice concentrate in Step (7) to high temperature instantaneous sterilization equipment, set the temperature to 125 degrees C, and obtain the sterilized juice concentrate.
(9) Obtain the sweetened composition: Perform spray drying for the sterilized juice concentrate, set the inlet air temperature to 165-170 degrees C and the outlet air temperature to 79-83 degrees C a, and obtain about 600 kg of dry powdery sweetened composition.

Through the detection of the content of components in the sweetened composition, the content of mogroside V is 38.0%, and the content of fructooligosaccharide is 24%.

### 1) Three-point flavor test

Respectively take the sweetened composition in the embodiment and the traditional mogroside product (from Guilin Layn Natural Ingredients Co., Ltd., with lot number of LHG211215-01, with the content of mogroside V of 39.3%), add purified water, and prepare into BRIX of about 40%. A three-point test was conducted, and the flavor was smelled and evaluated by six qualified sensory evaluators. The combinations are shown in the following table, where A represents the sweetened composition of the embodiment, and B represents the traditional mogroside product. The results showed that the sweetened composition of the embodiment was pure and had no obvious flavor, and the traditional mogroside product had a very obvious flavor of Momordica grosvenori. All evaluators could easily identify non-similar items from each combination.

| Sort by | AAB | ABA | BAA | BBA | BAB | ABB |
|---|---|---|---|---|---|---|
| Number of pairs | 6 | 6 | 6 | 6 | 6 | 6 |

### 2) Applications

A. Plant-based yoghurt: Take 0.1 part of the sweetened composition prepared in the embodiment, 95 parts of nut fermentation liquid, 4 parts of apple juice concentrate and 6 parts of Momordica grosvenori fruit concentrate, and mix.
B. Fresh fruit smoothie: Take 0.15 parts of the sweetened composition prepared in the embodiment, 12 parts of grapefruits, 15 parts of fresh green apple pulp, 10 parts of blueberries and 63 parts of whole milk, and homogenize.
C. Lip balm: Take 0.015 part of the sweetened composition prepared in the embodiment, 12 parts of beewax, 14 parts of vaseline, 33 parts of glycerin and 41 parts of tea seed oil, heat and melt, mix, mould and cool for molding.

The foregoing embodiments are descriptive only and the embodiments herein are demonstrated by the real test results of the applicant. Therefore, it is the intention of the applicant that the claims are not limited by the selection of examples describing the characteristics of the invention. Some numerical ranges used in the claims also include subranges, and variations in these ranges should also be interpreted to be covered by the claims where possible.

## Claims

1. A preparation method of a sweetened composition comprising up to 99% mogroside V and up to 99% fructooligosaccharide, **characterized by** including the following steps: take Momordica grosvenori extract, transfer the Momordica grosvenori extract into an enzyme membrane bioreactor, add fructosyltransferase or microorganism containing the fructosyltransferase, mix, perform enzymatic conversion treatment at a temperature of 40 to 50 degrees C and a pH of 3.5 to 6.0, obtain enzymatic conversion liquid on a membrane interception side, and obtain juice aqueous solution on a membrane permeation side;
The enzymatic conversion liquid is concentrated and sterilized to obtain the sweetened composition; or the juice aqueous solution is mixed with the enzymatic conversion liquid after sugar decomposition and isomerization, and the mixture is concentrated and sterilized to obtain the sweetened composition;
The sugar decomposition and isomerization include the following step: add sucrase and glucose isomerase to the juice aqueous solution for reaction at a pH of 3.0-8.0 and a temperature of 40-55 degrees C.

2. The preparation method of the sweetened composition according to claim 1, **characterized in that** the added content of the biological enzyme is measured by the added content of the fructosyltransferase, which is 0.1 to 0.5% of the total content of soluble solid matters in the Momordica grosvenori extract.

3. The preparation method of the sweetened composition according to claim 1, **characterized in that** the added content of the sucrase is 0.01-0.8% of the total content of the soluble solid matters in the juice aqueous solution, the added content of the glucose isomerase is 0.1-2.5% of the total content of the soluble solid matters in the juice aqueous solution, and the reaction is performed for 1-3 hours after the sucrase and the glucose isomerase are added.

4. The preparation method of the sweetened composition according to claim 1, **characterized in that** the Momordica grosvenori extract is obtained by extracting Momordica grosvenori with water and then filtering to remove impurities; the mass ratio of the water to the Momordica grosvenori is 2-5:1, the extraction temperature is 80-90 degrees C, and the extraction time is 20-40 minutes; the pore size of a filter membrane used in filtration is less than or equal to 1 µm.

5. The preparation method of the sweetened composition according to claim 1, **characterized in that** after-ripening treatment is required to be conducted on the Momordica grosvenori for 1-10 days before the Momordica grosvenori extract is obtained.

6. The preparation method of the sweetened composition according to claim 1 or 5, **characterized in that** the Momordica grosvenori extract is purified with resin before the Momordica grosvenori extract is transferred to the enzyme membrane bioreactor, and the resin comprises one or more of adsorbent resin, anion resin and cation resin; the enzymatic conversion liquid or juice aqueous solution is concentrated, sterilized and then dried to obtain the sweetened composition, and the drying treatment is belt drying or spray drying.

## Patentansprüche

1. Herstellungsverfahren einer gesüßten Zusammensetzung, die bis zu 99 % Mogrosid V und bis zu 99 % Fructooligosaccharid umfasst, **dadurch gekennzeichnet, dass** es die folgenden Schritte umfasst: Momordica-grosvenori-Extrakt bereitstellen, den Momordica-grosvenori-Extrakt in einen Enzym-Membran-Bioreaktor überführen, Fructosyltransferase oder einen die Fructosyltransferase enthaltenden Mikroorganismus zugeben, mischen, eine enzymatische Umwandlungsbehandlung bei einer Temperatur von 40 bis 50 °C und einem pH-Wert von 3,5 bis 6,0 durchführen, enzymatische Umwandlungsflüssigkeit auf einer Membranrückhalteseite erhalten und Saftwasserlösung auf einer Membranpermeationsseite erhalten;
die enzymatische Umwandlungsflüssigkeit wird konzentriert und sterilisiert, um die gesüßte Zusammensetzung zu erhalten; oder die Saftwasserlösung wird nach Zuckerzersetzung und Isomerisierung mit der enzymatischen Umwandlungsflüssigkeit gemischt, und das Gemisch wird konzentriert und sterilisiert, um die gesüßte Zusammensetzung zu erhalten;
die Zuckerzersetzung und Isomerisierung umfassen den folgenden Schritt: Sucrase und Glucoseisomerase zu der Saftwasserlösung zur Reaktion bei einem pH-Wert von 3,0-8,0 und einer Temperatur von 40-55 °C zugeben.

2. Herstellungsverfahren der gesüßten Zusammensetzung nach Anspruch 1, **dadurch gekennzeichnet, dass** die zugesetzte Menge des biologischen Enzyms anhand der zugesetzten Menge der Fructosyltransferase bemessen wird, die 0,1 bis 0,5 % des Gesamtgehalts an löslichen Feststoffen in dem Momordica-grosvenori-Extrakt beträgt.

3. Herstellungsverfahren der gesüßten Zusammensetzung nach Anspruch 1, **dadurch gekennzeichnet, dass** die zugesetzte Menge der Sucrase 0,01-0,8 % des Gesamtgehalts der löslichen Feststoffe in der Saftwasserlösung beträgt, die zugesetzte Menge der Glucoseisomerase 0,1-2,5 % des Gesamtgehalts der löslichen Feststoffe in der Saftwasserlösung beträgt, und die Reaktion 1-3 Stunden nach dem Zugeben der Sucrase und der Glucoseisomerase durchgeführt wird.

4. Herstellungsverfahren der gesüßten Zusammensetzung nach Anspruch 1, **dadurch gekennzeichnet, dass** der Momordica-grosvenori-Extrakt durch Extrahieren von Momordica grosvenori mit Wasser und anschließendes Filtrieren zum Entfernen von Verunreinigungen erhalten wird; das Massenverhältnis von Wasser zu Momordica grosvenori 2-5:1 beträgt, die Extraktionstemperatur 80-90 °C beträgt und die Extraktionszeit 20-40 Minuten beträgt; die Porengröße einer in der Filtration verwendeten Filtermembran kleiner als oder gleich 1 µm ist.

5. Herstellungsverfahren der gesüßten Zusammensetzung nach Anspruch 1, **dadurch gekennzeichnet, dass** vor dem Erhalt des Momordica-grosvenori-Extrakts eine Nachreifebehandlung an Momordica grosvenori für 1-10 Tage durchgeführt werden muss.

6. Herstellungsverfahren der gesüßten Zusammensetzung nach Anspruch 1 oder 5, **dadurch gekennzeichnet, dass** der Momordica-grosvenori-Extrakt mit Harz gereinigt wird, bevor der Momordica-grosvenori-Extrakt in den Enzym-Membran-Bioreaktor überführt wird, und das Harz eines oder mehrere von Adsorptionsharz, Anionenharz und Kationenharz umfasst; die enzymatische Umwandlungsflüssigkeit oder Saftwasserlösung konzentriert, sterilisiert und dann getrocknet wird, um die gesüßte Zusammensetzung zu erhalten, und die Trocknungsbehandlung Bandtrocknung oder Sprühtrocknung ist.

## Revendications

1. Procédé de préparation d'une composition édulcorée comprenant jusqu'à 99 % de mogroside V et jusqu'à 99 % de fructooligosaccharide, **caractérisé en ce qu'**il comprend les étapes suivantes : prendre un extrait de Momordica grosvenori, transférer l'extrait de Momordica grosvenori dans un bioréacteur enzymatique à membrane, ajouter une fructosyltransférase ou un microorganisme contenant la fructosyltransférase, mélanger, effectuer un traitement de conversion enzymatique à une température de 40 à 50 °C et à un pH de 3,5 à 6,0, obtenir un liquide de conversion enzymatique sur un côté de rétention de membrane, et obtenir une solution aqueuse de jus sur un côté de perméation de membrane;
le liquide de conversion enzymatique est concentré et stérilisé pour obtenir la composition édulcorée ; ou la solution aqueuse de jus est mélangée avec le liquide de conversion enzymatique après décomposition du sucre et isomérisation, et le mélange est concentré et stérilisé pour obtenir la composition édulcorée ;
la décomposition du sucre et l'isomérisation comprennent l'étape suivante : ajouter une sucrase et une isomérase du glucose à la solution aqueuse de jus pour une réaction à un pH de 3,0-8,0 et à une température de 40-55 °C.

2. Procédé de préparation de la composition édulcorée selon la revendication 1, **caractérisé en ce que** la quantité ajoutée de l'enzyme biologique est mesurée par la quantité ajoutée de la fructosyltransférase, qui est de 0,1 à 0,5 % du contenu total de matières solides solubles dans l'extrait de Momordica grosvenori.

3. Procédé de préparation de la composition édulcorée selon la revendication 1, **caractérisé en ce que** la quantité ajoutée de la sucrase est de 0,01-0,8 % du contenu total des matières solides solubles dans la solution aqueuse de jus, la quantité ajoutée de l'isomérase du glucose est de 0,1-2,5 % du contenu total des matières solides solubles dans la solution aqueuse de jus, et la réaction est effectuée pendant 1-3 heures après l'ajout de la sucrase et de l'isomérase du glucose.

4. Procédé de préparation de la composition édulcorée selon la revendication 1, **caractérisé en ce que** l'extrait de Momordica grosvenori est obtenu en extrayant Momordica grosvenori avec de l'eau puis en filtrant pour éliminer les impuretés ; le rapport massique de l'eau à Momordica grosvenori est de 2-5:1, la température d'extraction est de 80-90 °C, et le temps d'extraction est de 20-40 minutes ; la taille de pore d'une membrane de filtration utilisée dans la filtration est inférieure ou égale à 1 µm.

5. Procédé de préparation de la composition édulcorée selon la revendication 1, **caractérisé en ce qu'**un traitement de post-maturation doit être effectué sur Momordica grosvenori pendant 1-10 jours avant l'obtention de l'extrait de Momordica grosvenori.

6. Procédé de préparation de la composition édulcorée selon la revendication 1 ou 5, **caractérisé en ce que** l'extrait de Momordica grosvenori est purifié avec de la résine avant que l'extrait de Momordica grosvenori soit transféré dans le bioréacteur enzymatique à membrane, et la résine comprend une ou plusieurs parmi une résine adsorbante, une résine anionique et une résine cationique ; le liquide de conversion enzymatique ou la solution aqueuse de jus est concentré, stérilisé puis séché pour obtenir la composition édulcorée, et le traitement de séchage est un séchage sur bande ou un séchage par pulvérisation.
